# EUROPEAN PATENT APPLICATION

(11) **EP 3 882 614 A1**
(43) Date of publication of application: **22.09.2021**
(21) Application number: 19885183.4
(22) Date of filing: 13.11.2019
(51) Int. Cl.: G01N 27/26, G01N 21/64, G01N 33/543

(54) **BIOMOLECULE DETECTION DEVICE USING MICROPORES**

(30) Priority: 13.11.2018 KR 20180139200
(71) Applicant: Korea University Research and Business Foundation, Seoul 02841 (KR)
(72) Inventor: HAN, Chang Soo, Seoul 05236 (KR); BONG, Ki Wan, Seoul 04193 (KR); SHIN, Se Hyun, Seoul, 06574 (KR); RYU, Ji Hun, Seoul 02871 (KR); KIM, Hyeon Ung, Seoul 02857 (KR)
(74) Representative: Klunker IP Patentanwälte PartG mbB
(86) International application number: PCT/KR2019/015445
(87) International publication number: WO 2020/101347

(57) **Abstract**

The present invention relates to a biomolecule detection apparatus capable of easily and quickly detecting various biomolecules associated with diseases and determining the presence or absence of a specific disease. The biomolecule detection apparatus of the present invention includes a micropore device, a microchip, and sensing electrodes. According to the present invention, a microscale pore is formed inside the micropore device. In addition, the microchip is configured to pass through the microscale pore along the flow of a conductive liquid supplied inside the micropore device, has a surface coated with a sensing molecule complementarily bound to a target biomolecule, and has a unique code for identifying the complementarily bound target biomolecule. The sensing electrodes serve to sense the code by measuring change in current flowing through the pore when the microchip passes through the pore.

## Description

### [Technical Field]

The present invention relates to an inexpensive biomolecule detection apparatus using a micropore for detecting biomolecules that play an important role in disease metastasis or biomolecules as biomarkers.

### [Background Art]

Various methods for detecting DNA mutations are being studied to diagnose diseases such as cancers. In recent years, interest in non-invasive disease diagnosis technology capable of detecting mutations in cell-free DNA (cfDNA) circulating in human blood is increasing.

For this technology, it is very important to measure various disease signs simultaneously. Accordingly, non-invasive measurement methods at the biomolecular level have recently been developed. In this case, DNA, RNA, proteins, enzymes, and the like are used as major biomolecules.

Meanwhile, a conventional biomolecule detection apparatus detects a target biomaterial using a microchip including a sensing biomaterial that is complementarily bound to the target biomaterial. Specifically, only when the target biomaterial is complementarily bound to the sensing biomaterial provided in the probe region of the microchip, a light-emitting factor is selectively bound to a complex of the target biomaterial and the sensing biomaterial. Then, the microchip to which the target biomaterial is bound emits light in proportion to the concentration of the bound target biomaterial. At this time, by analyzing the coding information of the light-emitting microchip, the type of the detected target biomaterial can be determined.

In addition, in the case of the microchip, a code region for identifying the microchip can be determined by the wavelength (color) and shape of a fluorescent material. Accordingly, in the case of coding using the wavelength of a fluorescent material, due to limitation in distinguishable fluorescent colors, the number of codes is limited. In addition, since the fluorescent material for identifying the code region affects the analysis of a detection fluorescence signal for the target biological material, sensing sensitivity and specificity may deteriorate.

To solve these problems, a flow cytometer that can simultaneously identify fluorescence of the code region of a microchip and fluorescence of a probe region of the microchip may be used. However, the equipment is very expensive, and thus practical use of the equipment is limited.

In addition, to solve limiting factors in identifying a code region, a microchip may be coded using a geometric shape. However, to recognize the moving code region, an additional high-speed camera is required. In addition, a fluorescent material is included in the code region to simultaneously identify the code region and a probe region in the dark field. In this case, sensitivity and specificity may be degraded by the fluorescent material.

In addition, in the case of still photographing technology, the fluorescence of a probe can be identified in the dark field, and the shape of a code region can be identified in the bright field. However, image capture should be performed more than once, and editing of each image is required. Accordingly, a lot of time is consumed and a lot of effort is required. In addition, because automation is difficult, the measurement time is increased.

### [Disclosure]

### [Technical Problem]

Therefore, the present invention has been made in view of the above problems, and it is one object of the present invention to provide a biomolecule detection apparatus using a micropore. In the biomolecule detection apparatus, one side of a microchip complementarily bound to a target biomolecule to which a fluorescent material is bound is coated with DNA, and a unique identification code is formed on the other side thereof. With such a configuration, when a target biomolecule is bound to the microchip, the type of the target biomolecule may be determined by measuring the magnitude of a fluorescence signal generated from the unique code of the microchip, and based on this information, the presence or absence of a specific disease may be determined.

### [Technical Solution]

In accordance with one aspect of the present invention, provided is a biomolecule detection apparatus including a micropore device, a microchip, and sensing electrodes, wherein the micropore device has a microscale pore formed therein; the microchip is configured to pass through the pore along a flow of a conductive liquid supplied inside the micropore device, has a surface coated with a sensing molecule complementarily bound to a target biomolecule, and has a unique code for identifying the complementarily bound target biomolecule; and the sensing electrodes serve to sense the code by measuring change in current flowing through the pore when the microchip passes through the pore.

According to one embodiment, the microchip may be divided into a probe region having a surface coated with the sensing molecule and a coding region having the unique code for identifying the target biomolecule.

According to one embodiment, an entire surface of the microchip may be coated with the sensing molecule to form the probe region, and the code may be formed on a portion of the probe region.

According to one embodiment, the code may be formed in an uneven shape by cutting portions of both sides of the microchip in a predetermined pattern.

According to one embodiment, the code may be formed by forming a plurality of micro blow holes in the microchip and filling the micro blow holes with a conductive material in a predetermined pattern.

According to one embodiment, the code may be formed by forming a plurality of pattern layers having different porosities on one side of the microchip.

According to one embodiment, the code may be formed by coating a surface of one side of the microchip with a conductive material of a predetermined pattern.

According to one embodiment, to control flow of the conductive liquid flowing through the pore, the biomolecule detection apparatus may include a pressure pump or a flow control electrode.

According to one embodiment, the microchip may be formed using one or more selected from metal, ceramic, polymers, SiO₂, and hydrogel.

According to one embodiment, a plurality of blow holes having a porosity of 10 to 90 % may be formed inside the microchip.

According to one embodiment, the micropore device may be formed to have a thickness equal to or smaller than a length of a portion where the code of the microchip is formed.

According to one embodiment, the pore may be formed to have a diameter of 1 to 1,000 µm.

According to one embodiment, a fluorescent material may be bound to a surface of the target biomolecule, and the biomolecule detection apparatus may further include fluorescence signal detectors for detecting a fluorescence signal generated from the target biomolecule.

According to one embodiment, the fluorescence signal detectors may include a light source for emitting light to the target biomolecule complementarily bound to the sensing molecule, and a lens for receiving a fluorescence signal generated from the target biomolecule and obtaining a fluorescence image of the target biomolecule.

According to one embodiment, the lens may be configured as an optical microscope or a CCD camera.

In accordance with another aspect of the present invention, provided is a biomolecule detection apparatus including a micropore device, a microchip, a light source, a lens, and sensing electrodes, wherein the micropore device has a microscale pore formed therein; the microchip is configured to pass through the pore along a flow of a conductive liquid supplied inside the micropore device, is provided with a sensing molecule complementarily bound to a target biomolecule and having a surface to which a fluorescent material is bound, and has a unique code for identifying the complementarily bound target biomolecule; the light source is responsible for emitting light to the target biomolecule complementarily bound to the sensing molecule; the lens is responsible for receiving a fluorescence signal generated from the target biomolecule and obtaining a fluorescence image of the target biomolecule; and the sensing electrodes are responsible for sensing the code by measuring change in current flowing through the pore when the microchip passes through the pore.

### [Advantageous effects]

According to the present invention, when a target biomolecule to which a fluorescent material is bound is complementarily bound to a microchip, and the microchip is allowed to pass through a pore in the presence of an electrolyte, a fluorescence image of the target biomolecule complementarily bound to the microchip can be obtained by a lens. Based on the obtained fluorescence image, it can be confirmed whether the target biomolecule is complementarily bound to the microchip. Accordingly, the presence or absence of a specific disease can be confirmed in a measurement sample.

In addition, since a unique code for identifying a complementarily bound target biomolecule is formed on the microchip, by obtaining a fluorescence image and then sensing the code, based on the decoding information of the microchip, it can be confirmed that a target biomolecule includes a biomaterial associated with a specific disease.

In addition, when a biomaterial associated with a specific disease is detected, such detection information can be used as reference data necessary to diagnose the specific disease. Accordingly, cancers, genetic diseases, diabetes, and the like can be diagnosed early using a non-invasive method, and thus the present invention can contribute to disease prevention.

In addition, even when several types of target biomolecules are included in a biological sample, when the code of the microchip is formed differently according to the type of the target biomolecule, various types of target biomolecules can be simultaneously detected by identifying the code of the microchip.

In addition, since the type of a target biomolecule bound to the microchip can be determined without expensive equipment such as a flow cytometer, the present invention has economic advantages.

In addition, by individually measuring the microchip and simply measuring a fluorescence image at an on/off level, measurement accuracy can be improved. In addition, since the microchip passes quickly through the micropore device, compared to the prior art, measurement time can be reduced.

### [Description of Drawings]

FIG. 1 shows a configuration of a biomolecule detection apparatus according to one embodiment of the present invention.
FIG. 2 is a graph showing change in current flowing through a pore when the microchip shown in FIG. 1 passes through the pore.
FIG. 3 is a perspective view of the microchip shown in FIG. 1.
FIGS. 4 and 5 illustrate the probe region and the coding region of the microchip shown in FIG. 3.
FIGS. 5 to 8 illustrate microchips according to another embodiment, according to FIG. 1.
FIGS. 9 to 12 are graphs showing characteristics observed when a microchip with a coding region having another shape passes through a pore, according to FIG. 1.
FIG. 13 is a graph showing the intensity of a fluorescence signal depending on the number of target biomolecules bound to a sensing molecule.

### [Best Mode]

A biomolecule detection apparatus according to a preferred embodiment will be described in detail with reference to the accompanying drawings. In this specification, the same or similar elements are designated by the same reference numerals. In describing known functions and configurations, repeated description and description that may obscure the subject matter of the invention will be omitted. The embodiments of the present invention are provided to more fully describe the present invention to those skilled in the art. Therefore, the shape and size of elements in the drawings may be exaggerated for clearer explanation.

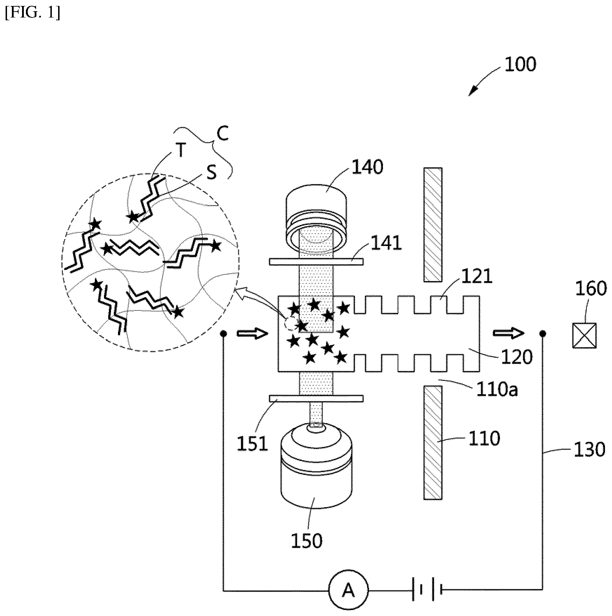

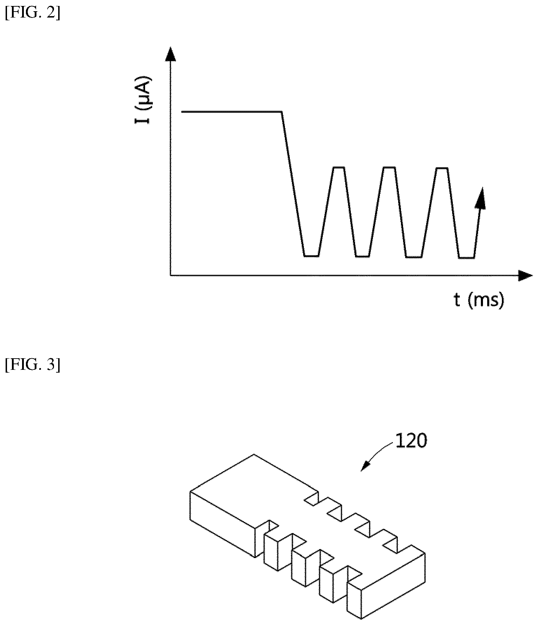

FIG. 1 shows a configuration of a biomolecule detection apparatus according to one embodiment of the present invention, FIG. 2 is a graph showing change in current flowing through a pore when the microchip shown in FIG. 1 passes through the pore, and FIG. 3 is a perspective view of the microchip shown in FIG. 1.

As shown in FIGS. 1 to 3, a biomolecule detection apparatus 100 includes a micropore device 110, a microchip 120, and sensing electrodes 130. In this case, movement of the microchip 120 through the micropore device 110 may be performed in a conductive liquid environment such as water or an electrolyte.

A microscale pore 110a may be formed in the micropore device 110. The micropore device 110 may be formed to have a thickness equal to or smaller than the length of a portion where a code 121 of the microchip 120 to be described later is formed. Specifically, the micropore device 110 may be formed by processing a membrane having a thickness of 20 µm or less, and the pore 110a may be formed to penetrate the membrane in the thickness direction of the membrane. The pore 110a is preferably formed to have a diameter of 1 to 1,000 µm, more preferably 10 to 200 µm.

In the present embodiment, the pore 110a is formed in a straight shape. However, the pore 110a may be formed in a tapered shape that widens or narrows in one direction. In addition, although not shown, a storage space for storing a conductive liquid may be formed in the micropore device 110. In this case, the conductive liquid contained in the storage space may be an electrolyte containing ions.

The microchip 120 may pass through the pore 110a along the flow of a conductive liquid flowing through the micropore device 110 by an external force such as pressure or an electric field. In addition, the microchip 120 may have a surface coated with a sensing molecule (S) complementarily bound to a target biomolecule (T). In this case, complementary binding means that specific bases bind to each other when DNA or RNA is synthesized. For example, when DNA is synthesized, adenine only binds to thymine, and guanine only binds to cytosine, which may be referred to as complementary binding.

In the present invention, the target biomolecule (T) may be any one of DNA, RNA, a protein, and an enzyme, and the sensing molecule (S) may be a material complementarily bound to DNA, RNA, a protein, or an enzyme. In addition, a state in which the target biomolecule (T) is complementarily bound to the sensing molecule (S) is indicated by (C) in the figure, and hereinafter the state is referred to as a complementary binding molecule (C).

Whether the complementary binding molecule (C) is detected may be confirmed by fluorescence signal detectors 140 and 150 to be described later, and detailed description thereof will be provided later.

Since the sensing molecule (S) complementarily bound to the target biomolecule (T) is bound to the surface of the microchip 120, even when the microchip 120 is immersed in a sample including various types of the target biomolecules (T), only one target biomolecule (T) complementarily bound to the sensing molecule (S) binds to the microchip 120, and thus only the desired target biomolecule (T) may be classified separately.

The microchip 120 may be formed using one or more selected from metal, ceramic, polymers, SiO₂, and hydrogel, which are materials that are not deformed in an electrolyte. In particular, when the microchip 120 is formed of SiO₂ or hydrogel, since blow holes are formed inside the microchip 120 due to the nature of the material, the overall weight of the microchip 120 may be reduced, and other materials may be loaded in the microchip 120.

A plurality of blow holes having a porosity of 10 to 90 % may be formed inside the microchip 120. The blow holes may be formed by artificially processing the microchip 120. Alternatively, as described above, the blow holes may be formed by forming the microchip 120 using SiO₂ or hydrogel.

On one side of the microchip 120, the unique code 121 for identifying the complementarily bound target biomolecule (T) may be formed. For example, as shown in FIG. 3, the code 121 may be formed in an uneven shape by cutting the portions of both sides of the microchip 120 in a predetermined pattern.

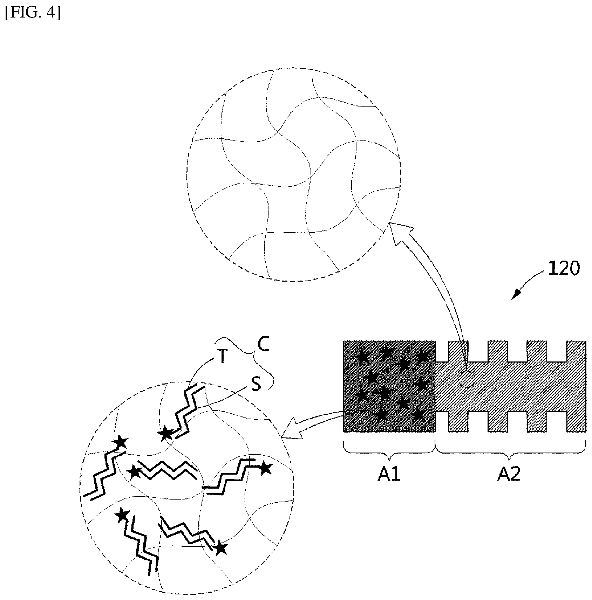

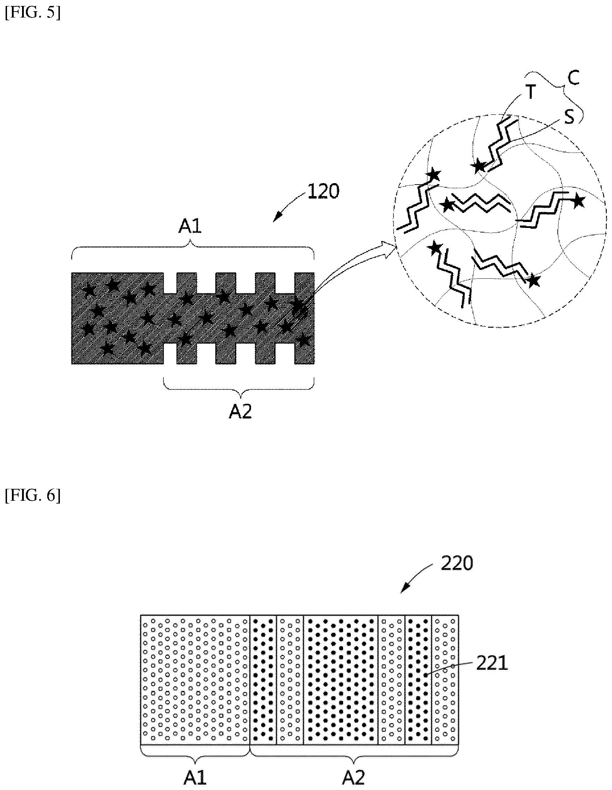

In addition, FIGS. 4 and 5 illustrate a probe region A1 and a coding region A2 of the microchip 120 shown in FIG. 3. As shown in FIG. 4, the microchip 120 may be divided into the probe region A1 having a surface to which the sensing molecule (S) is bound and the coding region A2 having the unique code 121 for identifying the target biomolecule (T). That is, since the sensing molecule (S) is bound to only the surface of the probe region A1, the target biomolecule (T) is bound to the probe region A1 and is not bound to the coding region A2.

In addition, as shown in FIG. 5, the sensing molecule (S) may be bound to the entire surface of the microchip 120 to form the probe region A1, and the unique code 121 for identifying the target biomolecule (T) may be formed on a portion of the probe region A1 to form the coding region A2. That is, the entire microchip 120 may correspond to the probe region A1, and the target biomolecule (T) may be bound to the entire microchip 120. To form the coding region A2, an uneven shape may be formed on a portion of the probe region A1 to which the target biomolecule (T) is bound. Formation of the probe region A1 and the coding region A2 is not limited to the illustrated example, and may be variously implemented.

The microchip 120 may be controlled to move in one direction. To control in this way, the micropore device 110 may include a pressure pump 160 or a flow control electrode (not shown) for controlling the flow of a conductive liquid flowing through the pore 110a, more specifically, an electrolyte. With this configuration, when the pressure pump 160 is driven, or when current is applied to the flow control electrode, an electrolyte flows in one direction, and the microchip 120 immersed in the electrolyte is allowed to move in the same direction as the flow of the electrolyte. In this case, the pressure pump 160 may be a syringe pump, and may be disposed on a flow path connected to the outlet of the micropore device 110.

The microchip 120 may be formed in a rectangular shape having a long length in one direction. When the length of the microchip 120 is longer than the diameter of the pore 110a through which the microchip 120 passes, when the microchip 120 immersed in an electrolyte passes through the pore 110a, the microchip 120 is aligned in the flow direction of the electrolyte, which enables more accurate signal measurement.

The sensing electrodes 130 may sense the code 121 by measuring change in current flowing through the pore 110a when the microchip 120 passes through the pore 110a. The sensing electrodes 130 may be disposed at the inlet and the outlet of the pore 110a, respectively, and may measure blocking current generated by the microchip 120 moving between the sensing electrodes 130.

That is, when the microchip 120 and a conductive liquid simultaneously pass through the pore 110a through which only an electrolyte has passed, the microchip 120 blocks the flow of current due to the movement of ions, so that potential difference between the inlet and the outlet changes. The sensing electrodes 130 measure the change in potential difference. Since the coding region A2 of an uneven shape is formed on the microchip 120, when the microchip 120 passes through the pore 110a, increase or decrease in potential difference may be repeated, and the sensing electrodes 130 may determine the size and shape of the microchip 120 by measuring change in the potential difference.

For example, potential difference in the microchip 120 shown in FIG. 1 may be expressed as in the graph shown in FIG. 2. That is, the number of peak values in the graph is determined according to the number of irregularities formed on the coding region A2 of the microchip 120, and the size and shape of the microchip 120 may be determined based on the shape and number of the peak values. By determining the shape of the microchip 120 through the graph created based on measurement by the sensing electrodes 130, the type of the target biomolecule (T) bound to the microchip 120 may be determined.

In addition, a fluorescent material may be bound to the surface of the target biomolecule (T), and the biomolecule detection apparatus 100 may further include the fluorescence signal detectors 140 and 150 for detecting a fluorescence signal generated from the target biomolecule (T). The fluorescent material is marked as a star (★) in the drawing.

The fluorescence signal detectors may be disposed at the inlet or outlet of the pore 110a, and may include a light source 140 and a lens 150.

The light source 140 may be disposed to be spaced apart from the microchip 120 by a predetermined distance and may emit light to the target biomolecule (T) complementarily bound to the sensing molecule (S). Accordingly, a fluorescence signal may be generated from the fluorescent material (★) bound to the target biomolecule (T). The light source 140 may be configured as one selected from a Mercury lamp, a laser diode (LD), and a laser light-emitting diode (LED). In this case, an excitation filtration filter 141 that filters only wavelengths generated from the fluorescent material (★) bound to the surface of the target biomolecule (T) may be disposed between the light source 140 and the microchip 120.

The lens 150 may be disposed on the opposite side of the light source 140 with respect to the microchip 120, and may receive a fluorescence signal generated from the fluorescent material (★) bound to the target biomolecule (T) and obtain a fluorescence image of the target biomolecule (T). The lens 150 may be configured as an optical microscope or a CCD camera. In this case, an emission filtration filter 151 that filters excitation light components and only wavelengths corresponding to the fluorescence signal may be disposed between the lens 150 and the microchip 120.

In this way, when the intensity of a fluorescence signal generated from the target biomolecule (T) bound to the microchip 120 is measured by the lens 150, the concentration of the target biomolecule (T) bound to the microchip 120 may be determined based on the intensity of each fluorescence, thereby enabling quantitative analysis. For example, when the concentration of the target biomolecule (T) measured by the lens 150 exceeds a certain range, it may be used as reference information for diagnosing the presence of a specific disease.

In the case of the microchip 120 in which the sensing molecule (S) and the target biomolecule (T) are not complementarily bound to each other, a fluorescence signal is hardly generated. Thereby, it may be determined whether the target biomolecule (T) is bound to the microchip 120.

Specifically, the lens 150 may detect the luminescence intensity of a fluorescent material bound to the target biomolecule (T) among the complementary binding molecules (C) bound to the microchip 120. Based on these measurement results, it may be determined whether a specific disease is present. When the luminescence intensity exceeds a certain range, it may be determined that a specific disease is present. When the luminescence intensity is less than a certain range, it may be determined that a specific disease is absent. The presence or absence of a specific disease may be expressed as "O" or "X" through a separate display.

As described above, in the biomolecule detection apparatus 100, when the target biomolecule (T) to which a fluorescent material is bound is complementarily bound to the microchip 120, and then the microchip 120 is allowed to pass through the pore 110a along the flow of an electrolyte, a fluorescence image of the target biomolecule (T) complementarily bound to the microchip 120 may be obtained by the lens 150. Based on the obtained fluorescence image, it may be determined that the target biomolecule (T) is complementarily bound to the microchip 120. Thus, it may be determined whether a specific disease is present in a sample to be measured.

In addition, since the unique code 121 for identifying the complementarily bound target biomolecule (T) is formed on the microchip 120, when a fluorescence image is obtained, and then the code 121 is sensed, based on the decoding information of the microchip 120, it may be determined whether the target biomolecule (T) includes a biomaterial associated with a specific disease.

In addition, when a biomaterial associated with a specific disease is detected, this results may be used as reference information for judging the presence or absence of the specific disease. Accordingly, by using a non-invasive diagnostic method, diseases such as cancers, genetic diseases, and diabetes may be detected early, thereby preventing aggravation of these diseases.

In addition, even when various types of the target biomolecules (T) are included in a biological sample, when the code 121 of the microchip 120 is formed differently according to the type of the target biomolecule (T), various types of the target biomolecules (T) may be simultaneously detected by identifying the code 121 of the microchip 120.

In addition, since the type of a target biomolecule bound to the microchip may be determined without expensive equipment such as a flow cytometer, the present invention has economic advantages.

In addition, by individually measuring the microchip 120 and simply measuring a fluorescence image at an on/off level, measurement accuracy may be improved. In addition, since the microchip 120 passes quickly through the micropore device 110, compared to the prior art, measurement time may be reduced.

FIGS. 6 to 8 show microchips according to another embodiment, according to FIG. 1.

As shown in FIG. 6, a code 221 may be formed in a microchip 220 by forming a plurality of micro blow holes in the microchip 220 and filling the micro blow holes with a conductive material in a predetermined pattern. In this case, the micro blow holes may be formed by processing the microchip 220. Alternatively, by forming the microchip 220 using SiO₂ or hydrogel, the micro blow holes may be naturally formed due to the characteristics of the material. In addition, the conductive material may include gold (Ag), silver (Au), and the like.

When the blow holes are filled with a conductive material in a predetermined pattern, the intensity of current at a portion filled with the conductive material increases. Accordingly, increase and decrease in the intensity of current measured by the sensing electrodes 130 are repeated according to the shape of the pattern. Thus, based on change in the intensity of current, the code 221 formed on the microchip 220 may be identified.

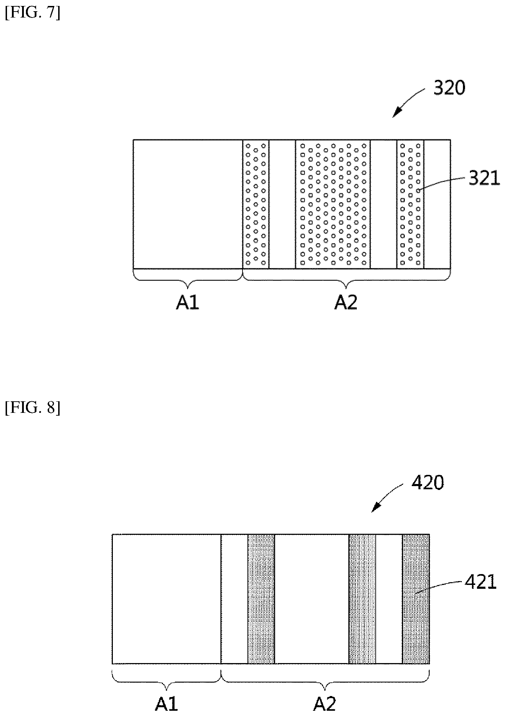

As shown in FIG. 7, a code 321 may be formed on a microchip 320 by forming a plurality of pattern layers having different porosities on one side of the microchip 320. When a plurality of pattern layers having different porosities is formed on the microchip 320 in this way, voids are filled with an electrolyte. Accordingly, increase and decrease in the intensity of current measured by the sensing electrodes 130 are repeated according to the shape of the pattern. Thus, based on change in the intensity of current, the code 321 formed on the microchip 320 may be identified.

As shown in FIG. 8, a code 421 may be formed on a microchip 420 by coating the surface of one side of the microchip 420 with a conductive material of a predetermined pattern. When the microchip 420 is coated with the conductive material in this way, the intensity of current at the coated portion increases. Accordingly, increase and decrease in the intensity of current measured by the sensing electrodes 130 are repeated according to the shape of the pattern. Thus, based on change in the intensity of current, the code 421 formed on the microchip 420 may be identified.

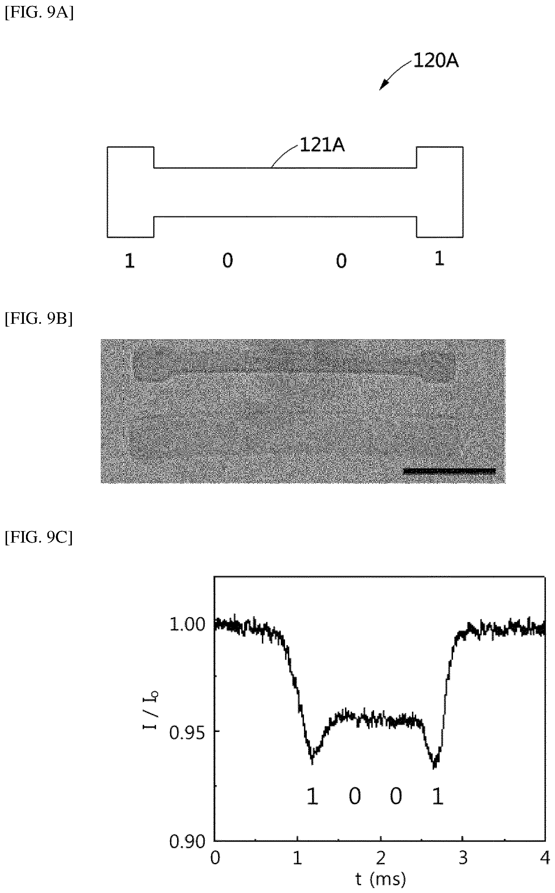

FIGS. 9 to 12 are graphs showing characteristics observed when a microchip with a coding region having another shape passes through a pore, according to FIG. 1. A method of detecting a biomolecule will be described with reference to FIGS. 9 to 12 as follows.

First, FIG. 9A schematically illustrates the microchip, FIG. 9B is an enlarged image of the actual microchip, and FIG. 9C is a graph showing change occurring when the microchip shown in FIG. 9B passes through a pore.

As shown in FIGS. 9A to 9C, a code 121A having two protrusions by forming one groove in the coding region A2 may be formed on a microchip 120A. In this case, a rectangular body may be formed to form the microchip 120A, and portions of both sides of the center of the body may be cut to form the code 121A of an uneven shape.

When the microchip 120A is fabricated in this way, a portion or the entire surface of the microchip 120A is coated with the sensing molecule (S) complementary bound to the specific target biomolecule (T), and then the microchip 120A is immersed in a biological sample. Accordingly, when the target biomolecule (T) is present in the biological sample, the target biomolecule (T) is bound to the microchip 120A.

The microchip 120A to which the target biomolecule (T) is bound may be moved to the micropore device 110 formed of a polyimide material along the flow of a conductive liquid. In this case, the conductive liquid may be prepared by adding an appropriate amount of 1 M KCl (final concentration: 1 mM KCl) to a solution containing DI water and PEG 600 in a ratio of 6:4. In addition, the pore 110a formed inside the micropore device 110 has a diameter of 67 µm and a thickness of 55 µm. The conductive liquid and the microchip 120A may pass through the pore 110a at a flow rate of 500 nl/s by negative pressure applied from a syringe pump.

In this case, since a fluorescent material is bound to the target biomolecule (T), when the microchip 120A passes through the pore 110a, a fluorescence image of the target biomolecule (T) may be captured using the lens 150. By analyzing the intensity of a fluorescence generated from the fluorescent material bound to the target biomolecule (T), it may be determined whether the target biomolecule (T) is bound.

When binding of the target biomolecule (T) is confirmed, a voltage of 50 mV is applied to the sensing electrodes 130 to measure blocking current generated when the microchip 120A passes through the pore 110a. The measured current is shown in FIG. 9C. That is, the number of peak values is determined according to the number of protrusions formed on the coding region A2 of the microchip 120A, and the shape of the microchip 120A may be identified through the number and shape of these peak values. Thus, in this way, the type of the target biomolecule (T) bound to the microchip 120A of a specific shape may be determined.

That is, when the intensity of luminescence generated from the target biomolecule (T) bound to the microchip 120 is measured by the fluorescence signal detectors 140 and 150, and based on these measurement results, the presence or absence of a specific disease is determined, the type of the target biomolecule (T) bound to the microchip 120 may be determined based on change in current measured by the sensing electrodes 130.

For example, when it is determined that the target biomolecule (T) bound to the microchip 120 has a mutation associated with a specific cancer, the sample may be determined to have the cancer. On the contrary, when a fluorescence signal is not detected by the fluorescence signal detectors 140 and 150, it may be determined that the target biomolecule (T) does not bind to the microchip 120, indicating that the sample has no disease factor.

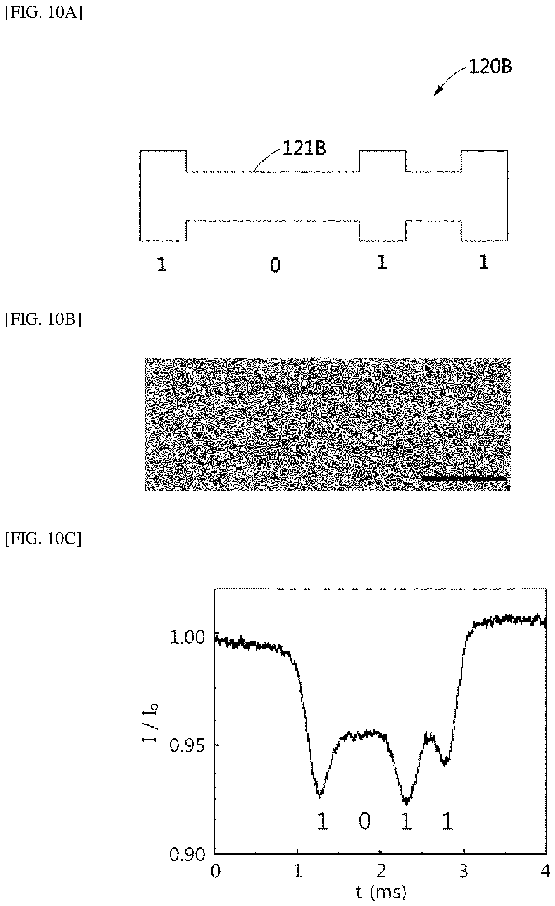

In addition, as shown in FIG. 10, when two grooves are formed in a code 121B of the coding region A2 of a microchip 120B, three protrusions are formed, and blocking current generated when the microchip 120B passes through the pore 110a is shown in the graph of FIG. 10C.

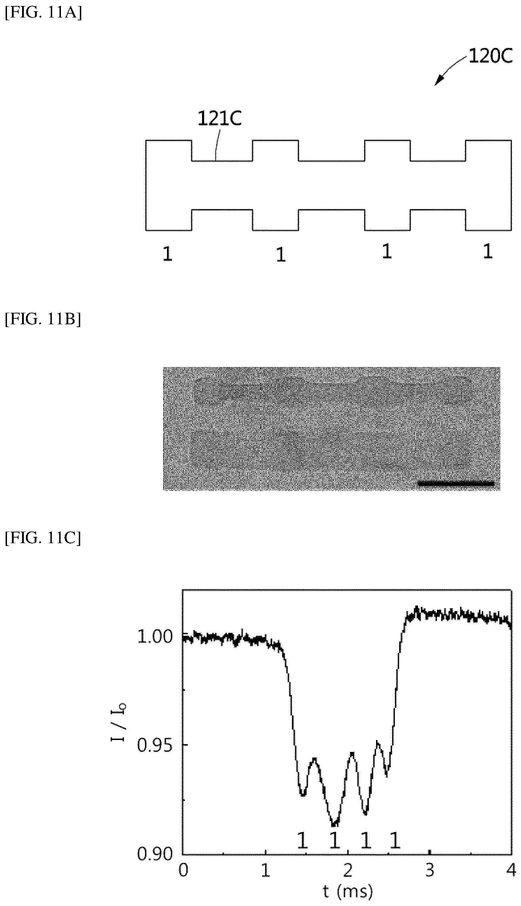

In addition, as shown in FIG. 11, when three grooves are formed in a code 121C of the coding region A2 of a microchip 120C, four protrusions are formed, and blocking current generated when the microchip 120C passes through the pore 110a is shown in the graph of FIG. 10C.

As described above, since graphs having different shapes are obtained according to the shape of the coding region of the microchip, the type of a target biomolecule bound to the microchip may be determined based on the shape of the graph.

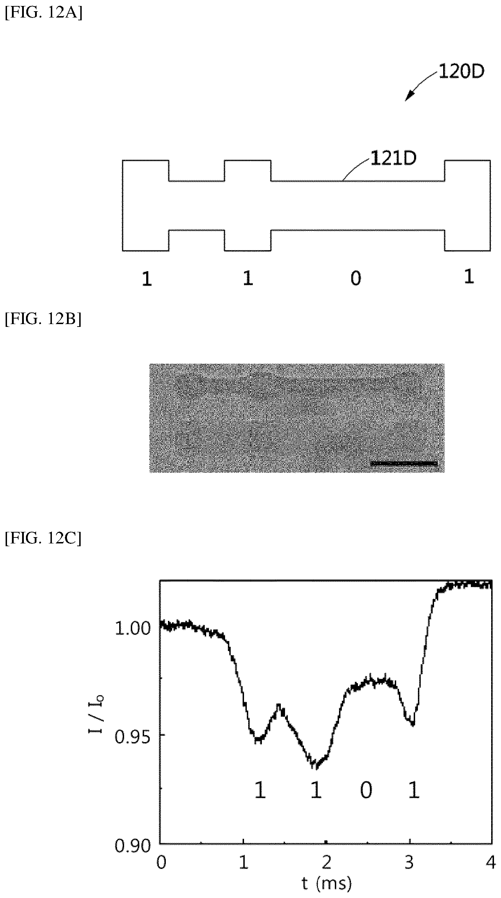

In addition, as shown in FIG. 12, by varying the position of protrusions formed in a code 121D of the coding region A2 of a microchip 120D, the shape of the microchip 120D may be determined.

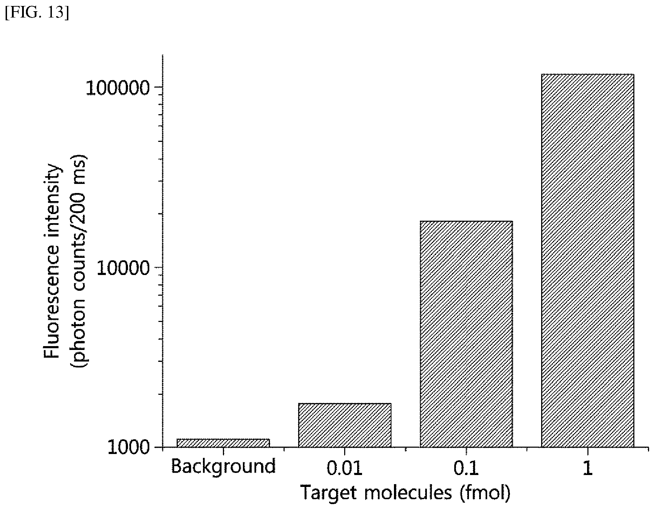

FIG. 13 is a graph showing the intensity of a fluorescence signal depending on the number of target biomolecules bound to a sensing molecule.

Referring to FIG. 13, it can be confirmed that, as the number of the target biomolecules (T) bound to the sensing molecule (S) increases, the intensity of a fluorescence signal measured by the fluorescence signal detectors 140 and 150 increases. Thus, based on the intensity of the measured fluorescence signal, the degree of infection of a disease may be determined.

Although the present invention has been described through limited examples and figures, the present invention is not intended to be limited to the examples. Those skilled in the art will appreciate that various modifications, additions, and substitutions are possible, without departing from the scope and spirit of the invention. Therefore, the scope of protection of the present invention should be defined by the following claims.

## Claims

1. A biomolecule detection apparatus, comprising:
a micropore device having a microscale pore formed therein;
a microchip configured to pass through the pore along a flow of a conductive liquid supplied inside the micropore device, having a surface coated with a sensing molecule complementarily bound to a target biomolecule, and having a unique code for identifying the complementarily bound target biomolecule; and
sensing electrodes for sensing the code by measuring change in current flowing through the pore when the microchip passes through the pore.

2. The biomolecule detection apparatus according to claim 1, wherein the microchip is divided into a probe region having a surface coated with the sensing molecule and a coding region having the unique code for identifying the target biomolecule.

3. The biomolecule detection apparatus according to claim 1, wherein an entire surface of the microchip is coated with the sensing molecule to form the probe region, and the code is formed on a portion of the probe region.

4. The biomolecule detection apparatus according to claim 1, wherein the code is formed in an uneven shape by cutting portions of both sides of the microchip in a predetermined pattern.

5. The biomolecule detection apparatus according to claim 1, wherein the code is formed by forming a plurality of micro blow holes in the microchip and filling the micro blow holes with a conductive material in a predetermined pattern.

6. The biomolecule detection apparatus according to claim 1, wherein the code is formed by forming a plurality of pattern layers having different porosities on one side of the microchip.

7. The biomolecule detection apparatus according to claim 1, wherein the code is formed by coating a surface of one side of the microchip with a conductive material of a predetermined pattern.

8. The biomolecule detection apparatus according to claim 1, wherein, to control flow of the conductive liquid flowing through the pore, the biomolecule detection apparatus comprises a pressure pump or a flow control electrode.

9. The biomolecule detection apparatus according to claim 1, wherein the microchip is formed using one or more selected from metal, ceramic, polymers, SiO₂, and hydrogel.

10. The biomolecule detection apparatus according to claim 1, wherein a plurality of blow holes having a porosity of 10 to 90 % is formed inside the microchip.

11. The biomolecule detection apparatus according to claim 1, wherein the micropore device is formed to have a thickness equal to or smaller than a length of a portion where the code of the microchip is formed.

12. The biomolecule detection apparatus according to claim 1, wherein the pore is formed to have a diameter of 1 to 1,000 µm.

13. The biomolecule detection apparatus according to claim 1, wherein a fluorescent material is bound to a surface of the target biomolecule, and the biomolecule detection apparatus further comprises fluorescence signal detectors for detecting a fluorescence signal generated from the target biomolecule.

14. The biomolecule detection apparatus according to claim 13, wherein the fluorescence signal detectors comprise a light source for emitting light to the target biomolecule complementarily bound to the sensing molecule, and
a lens for receiving a fluorescence signal generated from the target biomolecule and obtaining a fluorescence image of the target biomolecule.

15. The biomolecule detection apparatus according to claim 14, wherein the lens is configured as an optical microscope or a CCD camera.

16. A biomolecule detection apparatus, comprising:
a micropore device having a microscale pore formed therein;
a microchip configured to pass through the pore along a flow of a conductive liquid supplied inside the micropore device, provided with a sensing molecule complementarily bound to a target biomolecule and having a surface to which a fluorescent material is bound, and having a unique code for identifying the complementarily bound target biomolecule;
a light source for emitting light to the target biomolecule complementarily bound to the sensing molecule;
a lens for receiving a fluorescence signal generated from the target biomolecule and obtaining a fluorescence image of the target biomolecule; and
sensing electrodes for sensing the code by measuring change in current flowing through the pore when the microchip passes through the pore.
